# EUROPEAN PATENT APPLICATION

(11) **EP 4 006 546 A1**
(43) Date of publication of application: **01.06.2022**
(21) Application number: 20210774.4
(22) Date of filing: 30.11.2020
(51) Int. Cl.: G01N 33/50

(54) **APPLICATION OF STEM-CELL DERIVED MONOCYTES IN A MONOCYTE ACTIVATION TEST (MAT) FOR THE ASSESSMENT OF PYROGENICITY AND INFLAMMATORY POTENTIAL**

(71) Applicant: Medizinische Hochschule Hannover, 30625 Hannover (DE)
(72) Inventor: Lachmann, Nico, 31303 Burgdorf (DE); Moritz, Thomas, 53177 Bonn (DE); Spreitzer, Ingo, 65203 Wiesbaden (DE)
(74) Representative: Moré, Solveig Helga

(57) **Abstract**

The present invention relates to the field of testing for pyrogens, e.g., endotoxin /LPS, non-endotoxin pyrogen (NEP), process-related impurities or endogenous pyrogens. This is highly relevant, e.g., for quality testing and safety testing of pharmaceutical compositions. The invention provides a method of testing a composition for the presence of at least one pyrogen, comprising (i) incubating a human monocyte or macrophage population derived from pluripotent stem cells in vitro, e.g., a human CD45⁺/CD11b⁺/CD14⁺/CD34⁻/TRA1-60⁻ monozyte population or CD45⁺/CD11b⁺/CD14⁺/CD34⁻/TRA1-60⁻/CD163⁺ macrophage population derived from pluripotent stem cells with said composition, and (ii) determining a reaction of the monozytes in the population to the presence of the at least one pyrogen, preferably, determining the quantity of an inflammatory cytokine expressed by the monozytes. The invention also provides the use of such monozyte populations for testing a composition for at least one pyrogen, in essence, the use of monocytes derived from pluripotent stem cells for a monocyte activation test (MAT), and kits suitable for this assay. The use of such stem-cell derived monocytes overcomes difficulties raised e.g., by variability of donor cells and allows for test systems that are reproducable and stable in the long term.

## Description

The present invention relates to the field of testing for pyrogens, e.g., endotoxin /LPS, non-endotoxin pyrogen (NEP), process-related impurities or endogenous pyrogens. This is highly relevant, e.g., for quality testing and safety testing of pharmaceutical compositions. The invention provides a method of testing a composition for the presence of at least one pyrogen, comprising (i) incubating a human monocyte or macrophage population derived from pluripotent stem cells in vitro, e.g., a human CD45⁺/CD11b⁺/CD14⁺/CD34⁻/TRA1-60⁻ monozyte population or CD45⁺/CD11b⁺/CD14⁺/CD34⁻/TRA1-60⁻/CD163⁺ macrophage population derived from pluripotent stem cells with said composition, and (ii) determining a reaction of the monozytes in the population to the presence of the at least one pyrogen, preferably, determining the quantity of an inflammatory cytokine expressed by the monozytes. The invention also provides the use of such monozyte populations for testing a composition for at least one pyrogen, in essence, the use of monocytes derived from pluripotent stem cells for a monocyte activation test (MAT), and kits suitable for this assay. The use of such stem-cell derived monocytes overcomes difficulties raised e.g., by variability of donor cells and allows for test systems that are reproducable and stable in the long term.

Pyrogen detection is of utmost importance in the pharmaceutical industry, laboratories and health care institutions. Adverse reactions to parenteral preparations have been described as early as the late 19th century. The agents capable of inducing such fever are termed "pyrogens". It turned out that the capability of inducing fever is distinct from sterility, as, for example, components of the cell wall of gram-negative bacteria, lipopolysaccharids (LPS) or endotoxins, were also found to be pyrogenic if the bacteria are dead. Due to their stability, endotoxins can be very difficult to remove by classical bactericidal procedures such as heating or filtration. This makes control of the whole production process necessary.

Hort and Penfold, in 1912, were the first to design a pyrogen test, which was based on injection into rabbits, and analysis of the increased in body temperature of the rabbits. This pyrogen test was included in the 12th edition of the United States Pharmacopoeia (USP) in 1942. The rabbit pyrogen test is still in use today in some cases, but it is costly, time consuming, and problematic due to the use of animals (Vipond et al., 2016. ALTEX).

The first and most successful alternative test was the bacterial endotoxin test (BET) based on the lysate of amoebocytes from the blood of horseshoe crabs, the limulus amebocyte lysate (LAL) assay, which became commercially available in the 1970s and has been widely used as a replacement for the rabbit pyrogen test. However, because it only detects LPS endotoxins, other pyrogenic materials are missed. Also, certain conditions (sub-optimal pH conditions or unsuitable cation concentration) can lead to false negatives. Glucans from carbohydrate chromatography matrices can also lead to false positives.

Poole et al. in 1988 introduced an assay of pyrogenic contamication in pharmaceuticals by cytokine release from monocytes (Poole et al. 1988. Dev Biol Stand 69:121-123). Since January 2010, the Monocyte Activation Test has been described as a compendial method for pyrogen detection in the European Pharmacopeia (chapter 2.6.30) and since the 2016 revision, recommendations have been given to replace tests on rabbits with the Monocyte Activation Test (MAT), wherever possible and after product specific validation (EP 2.6.8, Rev. July 2016) (MerckMillipore, 2017 White Paper: Monocyte Activation Test (MAT), MerckKGaA, Darmstadt). The MAT is widely accepted, however, several disadvantages remain. The use of primary cells is not only time-consuming and labour-intensive, but it also leads to a high variability between tests. Even use of the same donor over time leads to different results, as the monocytes change over time. To overcome this, pools of macrophages from different subjects are sometimes used, but real stability cannot be obtained by this approach.

Use of monocyte cell lines for the MAT was also already suggested by Poole et al. 1988. If cell lines are employed, the variability between assays using the same cell line is lower, but still not optimal. Further, the limited availablity of monocyte cell lines leads to a small spectrum of genetic backgrounds of monocytes or macrophages, which are further all malignant cells. This has the consequence that the assay may not be representative in all situations.

In light of the state of the art, the inventors addressed the problem of providing an advantageous method and suitable cell populations for testing a composition, e.g., a pharmaceutical composition, for the presence of at least one pyrogen. Some or all of the above-mentioned problems are solved by the present invention, in particular, by the subject matter of the claims. Advantageously, a test system for pyrogens stable for at least decades is provided.

The present invention thus provides a method of testing a composition for the presence of at least one pyrogen, comprising
(i) incubating a human CD45⁺/CD11b⁺/CD14⁺/CD34⁻/TRA1-60⁻ monozyte population derived from pluripotent stem cells with said composition, and
(ii) determining a reaction of the monozytes in the population to the presence of the at least one pyrogen, preferably, determining the quantity of an inflammatory cytokine expressed by the monozytes.

Accordingly, the invention also provides the use of a human monozyte population comprising CD45⁺/CD11b⁺/CD14⁺/CD34⁻/TRA1-60⁻ monozytes derived from pluripotent stem cells for testing a composition for the presence of at least one pyrogen, in particular, using the method of the invention. Preferably, said testing for the presence of at least one pyrogen is for quality control and/or safety control of said composition. Quality control means testing if a composition complies with specific standards, e.g., if it has a desired potency or activity, in particular, a desired pyrogenic or pro-inflammatory potency or activity. The desired pyrogenic or pro-inflammatory potency may be absence of such activity, but, depending on the product, as further explained below, a specific pyrogenic or pro-inflammatory potency may also be desired. The composition may also be tested for a specific anti-inflammatory potency.

The methods of the invention may also be used for characterisation, in particular, when it is of interest to determine the properties of a composition, e.g., for providing a pyrogenic profile of a product or composition not yet characterised. For example, it can be tested which pyrogens ae responsible for a pyrogenic response, e.g., using inhibitors of specific Toll like receptors or genetically modified cells as described below.

The assay of the invention can be advantageously used in the context of quality control, testing toxicity, release of charges of a pharmaceutical composition, diagnostics and research, e.g., in preclinical research.

The monocytes may be used in the method of the invention at different differentiation stages. They may be CD163⁻ monocytes, or they may be CD163+ macrophages.

The inventors have surpirsingly found that, using monocytes or macrophages derived from pluripotent stem cells leads to a better reproducability of the data. Variations between the tests are extremely low. Further, advantageously, if the test is carried out with monocytes or macrophages derived from pluripotent stem cells, e.g., iPSC-macrophages, it was found to allow for a higer dynamic range, i.e., it can detect lower and higher concentrastions of pyrogens.

The at least one pyrogen may be
- LPS (lipopolysaccharide, i.e., the main constituent of the outer cell wall of gram-negative bacteria). LPS is also designated endotoxin;
- a non-endotoxin pyrogen (NEP). NEPs are typically MAMPs (Microbe associated molecular patterns) or PAMPs (Pathogen associated molecular patterns). Examples for a NEP are flagellin, peptidoglycan, lipoprotein, lipoteichoic acid, FSL1 (Fibroblast-stimulating lipopeptide 1, a synthetic diacylated lipoprotein that activates the TLR2/TLR6 heterodimer), MALP2 (Macrophage-activating lipopeptide-2, also a TLR2/6 agonist), a viral pyrogen (e.g., virion components from myxoviruses such as influenzy), a yeast pyrogen and a fungal pyrogen (e.g., capsular polysaccharide);
- a product-related impurity. Typical product-related impurities are host-cell proteins, host-cell DNA or the bioburden (i.e., residue from microbiological contamination);
- a process-related impurity or pyrogenic chemical agent. Examples for pyrogenic chemical agents, which may be comprises in a composition are polyadenylic, polyuridylic, polybionosinic and polyribocytidylic acids, or uncoupling agents of oxidative phosphorylation (Picric acid (trinitrophenol), dinitrophenol or 4,6-dinitro-o-cresol), N-phenyl-β-naphthylamine, Aldo-α-naphthylamine, metals such as nickel salts or disruptors of the thermoregulatory centers (LSD, cocaine, morphine) (Borton et al., 2018, ALTEX). Further, pyrogenic process-related impurities such as particles, e.g., small enough for being phagocytosed such as nanoparticles (1 nm to less than 1 µm) may be contained. For example, rubber particles, microscopic plastic particles, organic dust, or metal compounds in elastomers may be pyrogenic process-related impurities. Particles used as drug carriers, e.g., nanoparticles, may also bind large amounts of endotoxin, and may thus have a pyrogenic effect;
- a damage-associated molecular pattern (DAMP, also: danger-associated molecular pattern). In contrast to pathogen-associated molecular patterns (PAMPs), DAMPs are host biomolecules that can initiate and perpetuate a noninfectious inflammatory response. For example, they are released from damaged or dying cells and activate the innate immune system by interacting with pattern recognition receptors (PRRs). Many DAMPs are nuclear or cytosolic proteins with defined intracellular function that, when released outside the cell after tissue injury, move from a reducing to an oxidizing milieu resulting in their functional denaturation. Further to those DAMPs, there are other DAMPs originated from different sources, such as ECM (extracellular matrix), mitochondria, granules, ER (endoplasmic reticulum), and plasma membrane. Exemplary DAMPs are: byglycan, decorin, versican, LMW hyaluronoan, heparan sulfate, fibronectin, or the EDA domain thereof, tenascin, uric acid, S100 proteins, ATP, F-actin, cyclophilin A, Aβ, histones, HMGB1, HMGN1, IL-1α, IL-33, SAP130, DNA (e.g., CpG nucleotides), RNA, mtDNA, TFAM, Formyl peptide, mROS, calreticulin, defensins, cathelicidin (LL37), EDN (eosinophil-derived neurotoxin), granulysin, syndecan, and glypican, heat shock proteins (HSP such as HSP60, HSP70 or gp96) (Wikipedia); or
- a combination thereof.

The at least one pyrogen may also be an endogenous pyrogen, e.g., a cytokine, chemokine or other messenger produced by the body itself as a reaction to contact with exogenous pyrogens or DAMPs, e.g., IL-1, IL-6, IL-12, TNF-α or MCP1. Such cytokines or messengers may be human, but cytokines or messengers from animals may also be cross-reactive, and induce fever in humans.

Typically, the composition that is tested for the pyrogen is
(I) a pharmaceutical composition selected from the group comprising a plasma-derived pharmaceutical composition, a vaccine and another composition for injection or implantation (e.g., comprising a (optionally, new) active agent or lead compound or derivate that is tested for the purpose of finding out if it has a pyrogenic activity, which will typically not be desired), or
(II) a composition obtained from rinsing a medical device, or the composition comprising the medical device, or
(III) reference material for a diagnostic kit.

In most pharmaceutical compositions, in particular, if they are intended for injections, pyrogens are not desired, or the concentration of pyrogens must be below a specific standard. They must thus be tested for an eventual content of pyrogens for quality control and/or safetety control purposes. Pyrogens may e.g., be present in such compositions because of bacterial contaminations, as a left-over from the production process, e.g., if the composition comprises a recombinant protein or other bacterial product, or a DAMP as a contamination due to production in eukaryotic cells. Pharmaceutical compositions, e.g., plasma-derived pharmaceutical compositions are further sometimes contaminated with process-related impurities, which may e.g. be derived from leukapheresis or other blood-separation techniques, or product-related impurities, e.g., derived from host cells in which a recombinant protein has been produced.

A composition for implantation may, e.g., be a delayed release form of a pharmaceutical composition. therapeutic compositions may e.g., be therapeutic compositions, diagnostic compositions are compositions for preventing a disease or condition or reducing effects thereof (e.g., a vaccine).

Similarly, medical devices have to be tested for their pyrogenic potential, wherein a composition obtained from rinsing a medical device (e.g., with water or a buffer) is tested for the presence of pyrogens (e.g., based on European Pharmacopeia 3.3.4 Plastic containers for human blood; pyrogen test with rinsing solution, or with a comparable test). Alternatively, the MAT also allows for direct incubation with the tested medical device (or synonymously, for incubation with a composition comprising the medical device), so that material-mediated pyrogenicity can be directly analysed. In that case, the composition to be tested comprises the medical device, at least for a defined time, e.g., during co-incubation with the monozyte population.

In most settings, pyrogens are not desired, i.e., the method of the invention is carried out for quality control or for controlling toxicity of said composition, or, if the composition is derived from wahshing a medical device, of said device. Accordingly, if the content of pyrogen is too high, e.g., higher than a previously determined standard, the composition or medical device is to not to be used pharmaceutically.

A reference material for a diagnostic kit may e.g., be a pyrogen standard, e.g., an endotoxin or NEP, as described above.

Vaccines typically comprise at least one adjuvant that acts as a pyrogen, and thus helps in activating the immune system and priming of a response to the antigen(s) contained in the vaccine. A pyrogenic effect may thus be intended in a vaccine. However, to avoid a toxic or suboptimal response, the quantity and, optimally, quality of said pyrogenic response should be controlled, which can advantageously be carried out with the method of the invention. Similarly, the method of the invention may also be used to test pharmaceutical compositions, or active agents or adjuvants therof that inherently have a pyrogenic effect, e.g., the pyrogenic cytokines or messengers disclosed herein, or adjuvants.

Thus, the at least one pyrogen may also be a pharmaceutical composition that is to be tested for its inflammatory or anti-inflammatory potency. Thus, subpotent or hyperpotent charges or intermediates in production of a pharmaceutical composition may be identified and, as appropriate, eliminated, diluted, concentrated or further purified.

A pharmaceutical composition has an inflammatory potency if it acts as a pyrogen. It has an anti-inflammatory potency if it inhibits or reduces the pyrogenic affect of a standard composition having a known pyrogenic effect. Examples of active agents with anti-inflammatory potency are antibodies to any of the pro-inflammatory cytokines disclosed herein, e.g., anti-TNF-a, anti-IFNα, anti-IL-1α, anti-IL-8, anti-MCP1, etc., or blocking antibodies to a Toll-like receptor, e.g., to TLR-2, TLR-6 or TLR-9.

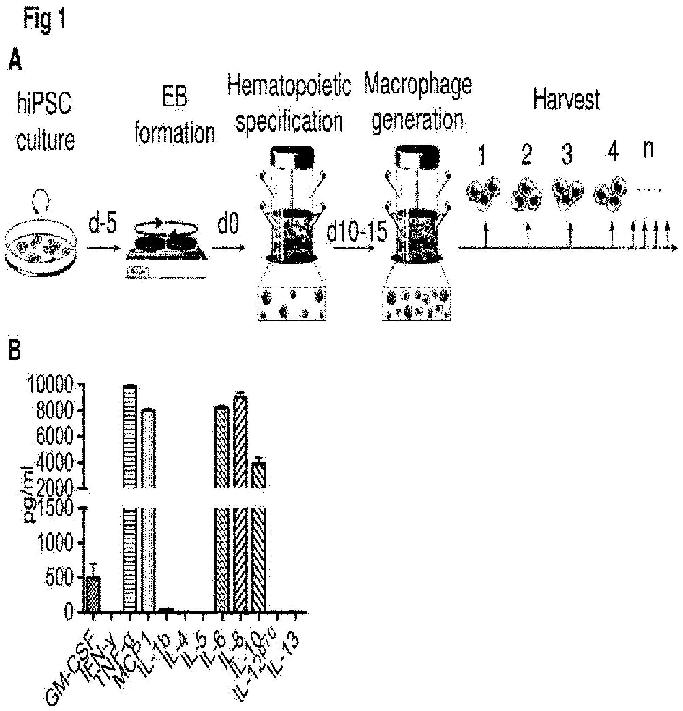

In step (i), the composition that is to be tested may be undiluted or titrated to allow for detection even if a great amount of pyrogen is comprised. Examples for the range of pyrogen concentrations that can be detected are provided in Fig. 1.

The composition is contacted with the monozyte population, wherein the monozytes are, e.g. present in a cell number of about 1*10⁴ to about 5*10⁴ cells per vial for the incubation of step (i). The incubation may be carried out, e.g., for about 1 hour to three days, e.g., for at least 4 hours to 1 day, or preferably, 6-12 hours or over night. The incubation is typically done under cell culture conditions, i.e., 37°C, 5% CO₂. Flat-bottom plates are typically used. The supernatant from the incubation may be frozen before further analysis.

The MAT test can, except for the cell population used, e.g., be carried out as described in US 2010/0203551 A1, in Carlin and Viitanen (2003. Pharmeuropa 15, 3:418-423), Nakagawa et al. 2002. Clinical and Diagnostic Laboratory Immunol. 9, 3:588-597) or Yamamoto et al. 2003. Jpn. J. Infect. Dis., 56. 93-100). For detection of specific pyrogens, appropriate plastic material is preferably used.

### Origin of monocyte populations

Optionally, the monozyte population employed for the invention is derived from at least one healthy subject. Alternatively, it can be employed from at least one subject having a condition selected from the group comprising an allergy, an auto-immune disease, an infection, a hereditary disease and a cancer. The subject may also have a certain age, e.g., the monozyte population may be derived from a subject of an age of 0-2 years, 2-10 years, 10-20 years, 20-30 years, 30-40 years, 40-50 years, 50-60 years, 60-70 years, 70-80 years, 80-90 years or older than 90 years, or a certain ethnicity, or a certain sex, i.e, male or female.

One of the stengths of the method of the invention is that it can easily be adapted to test monocyte populations derived from a plurality of subjects, and thus, from a plurality of pluripotent stem cells. This is not possibly for monocyte or macrophage cell lines, as these typically require different cell culture conditions. Using monocyte populations derived from a plurality of subjects has the advantage that a plurality of genetic backgrounds and/or conditions can be taken into account. Reactions to pyrogens may depend on the genetic background, sex, age and/or on conditions, e.g., diseases of the subject that is contacted with the pyrogen, or from which the stem cells are derived.

Further, if monocyte populations derived from a plurality of subjects are used, variability between different tests using monocytes derived from different stem cells may be lower. The results may also be more meaningful, as they may better reflect the reaction of different subjects to the composition.

In one embodiment of the invention, the monozyte population is thus derived from a plurality of subjects. Accordingly, it is derived from a plurality of pluripotent stem cells from different subjects. Pooling may take place at any step, e.g., the stem-cells may be pooled, myeloid cell forming complexes may be pooled, or the populations may be pooled after differentiation into monocytes. The plurality may comprise at least two, at least three, at least five, at least 10, at least 20, at least 25, at least 50, at least 100 or at least 200 different subjects. Preferably, the monocyte population is derived from at least four subjects, e.g., 4-10 subjects 6-8 subjects. This embodiment has the advantage that handling is easy, and the method is labour-saving. The test can be carried out, in essence, as if the monocytes were derived from only one subject. It can thus be advantageously be used, e.g., for routine applications such as in quality control.

In an alternative embodiment, a plurality of human CD45⁺/CD11b⁺/CD14⁺/CD34⁻/TRA1-60⁻ monozyte populations derived from pluripotent stem cells are separately incubated with said composition that is to be tested, and the reaction is separately determined according to step (ii) of the method of the invention. Said plurality of populations is derived from a plurality of subjects. The results may be analysed to produce an average and/or median result. The plurality may comprise at least two, at least three, at least five, at least 10, at least 20, at least 25, at least 50, at least 100 or at least 200 different monocyte populations derived from different subjects. Preferably, a plurality of 4-10, e.g., 6-8 monocyte populations is used. This embodiment allows for a more detailed data analysis, as results for different subjects can be separately analysed as well as together. It the results are analysed together, e.g., in the form of average or median results, statistic data is also available, which may allow, e.g., a prediction on the percentage of subjects that may be treated with the composition that may react to a certain composition in a certain manner, e.g., with fever or a strong and potentially dangerous inflammatory response. Further, if the results are analysed seperately, additional date may also be available, which may potentially allow for an analysis of reasons of or correlations with exceptional results. This embodiment may thus be particularly suitable if undesired pyrogenic reactions to a composition have already been noted, or for research purposes.

In either embodiment, the monozyte population or the plurality of monozyte populations may be derived from a plurality of healthy subjects.

The monozyte population or the plurality of monozyte populations may also be derived from a plurality of subjects all sharing at least one common characteristic selected from the group comprisig age (e.g., an age of 0-2 years, 2-10 years, 10-20 years, 20-30 years, 30-40 years, 40-50 years, 50-60 years, 60-70 years, 70-80 years, 80-90 years or older than 90 years), sex (male or female), ethnicity (e.g., Caucasean, Asian, African, African American, etc.), and/or each havig a condition, e.g., an allergy, an auto-immune disease, an infection, a hereditary disease and a cancer. The common characteristic may also be a mutation or a specific allele potentially affecting the immune response, in particular, the innate immune response, e.g., the sensitivity to allergy, auto-immunity or to development of sepsis. The common charcacteristic may be a comon MHC allele. More than one characteristic may be shared, e.g., 2, 3, 4, 5, 6 or 7 characteristics.

Optionally, the results obtained based on monocytes derived from stem cells of subjects sharing at least one characteristic are compared with monocytes derived from stem cells of subjects that do not have said characteristic. Thus, it can be tested if said characteristic affects the response of the monocytes to the pyrogen.

Optionally, the subjects share further characteristics. Alternatively, they may be selected to differ in regard to further characteristics to increase the chances that differences are due to the characteristic that is being analysed.

The subjects can also be selected to share at least one characteristic that may be associated with a specific disease or condition for which the pharmaceutical composition or medicinal product is indicated. Thus, the analysis would lead to results still more relevant for the group of subjects or patients concerned.

### Preparation of monocytes

The monocytes employed in the invention may be prepared from pluripotent stem cells in any manner, e.g., as known in the art.

They may for example be derived from embryonic stem cells. Alternatively, they may be derived from induced pluripotent stem cells (iPSC).

Methods for producing monocytes, and, optionally, CD163+ macrophages from pluripotent stem cells by means of adherent culture e.g., as described in example 2 below, or by methods known in the art (e.g., Lachmann et al., 2014 Am J Crit Care Respir Med, Lachmann et al. 2015 Stem Cell Reports, Ackermann et al., 2017 Transfus Med Hematother, Neehus et al., 2018 Stem Cell Reports).

In one preferred embdoiment, the monocyte population is obtainable from pluripotent stem cells by means of suspension culture. This has the advantage that great numbers of cells can be produced in an easy manner.

For example, the monozyte population may be obtainable from a method of producing myeloid cells, preferably, monozytes, said method comprising steps of
a) cultivating embryoid bodies in the presence of IL-3 and, optionally, M-CSF and/or GM-CSF, for a sufficient period of time to produce myeloid cell forming complexes;
b) cultivating the myeloid cell forming complexes in the presence of IL-3 and, preferably, M-CSF and/or GM-CSF for a sufficient period of time to produce myeloid cells preferably, monozytes; and
c) isolating said myeloid cells, preferably, monozytes,
d) and, optionally, freezing said myeloid cells, and thawing and washing said myeloid cells,
   wherein, if the isolated cells were not cultivated in the presence of M-CSF and/or GM-CSF in steps a) and/or b), they are cultivated in the presence of M-CSF and/or GM-CSF after isolation to provide monozytes.

If macrophages are used, the macrophage population employed is obtainable from a method of producing myeloid cells producing macrophages, comprising steps of
a) cultivating embryoid bodies in the presence of IL-3 and, optionally, M-CSF, for a sufficient period of time to produce myeloid cell forming complexes;
b) cultivating the myeloid cell forming complexes in the presence of IL-3 and, optionally, M-CSF, for a sufficient period of time to produce myeloid cells; and
c) isolating the macrophages.

Step a) is preferably carried out in in suspension culture. It may also be carried out in adherent culture. Step b) is preferably carried out in in suspension culture. Preferably, both step a) and b) are carried out in suspension culture.

Said method allows for continuous production, and, preferably, it is used for continuous production of myeloid cells. Preferred methods are disclosed in WO 2018/202881 A1, which is fully incorporated herein by reference.

Preferably, the embryoid bodies of the invention are obtained by a method comprising cultivating pluripotent stem cells such as iPSC, in suspension culture or in adherent cultur, for a sufficient period of time to produce embryoid bodies. The use of feeder cells may be avoided. This can be compensated for by adding cytokines e.g. SCF, BMP4, VEGF or small molecules including WNT pathway modulators such as CHIR99021 ((6-[[2-[[4-(2,4-Dichlorophenyl)-5-(5-methyl-1H-imidazol-2-yl)-2-pyrimidinyl]amino] ethyl]amino]-3-pyridinecarbonitrile (TOCRIS)) and BIO ((2'Z,3'E)-6-Bromoindirubin-3'-oxime) (TOCRIS)). Preferably, the culture conditions include use of ROCK inhibitor, but addition of bFGF is not required.

In a preferred embodiment, the suspension culture is carried out in a bioreactor allowing suspension culture such as an Erlenmeyer flask, spinner flask, stirred tank bioreactor, wave bioreactor and rotating wall bioreactor, preferentially in a stirred tank bioreactor, most preferably in an instrumented stirred tank bioreactor, i.e., a bioreactor equipped with technologies to monitor and control process parameters such as pH, pO2, temperature and stirring speed. Such systems are scalable, i.e., the volume of the culture can be changed without significantly changing culture conditions. The DASbox Mini bioreactor system, Eppendorf may be used, e.g., as described below.

For adherent culture, any tissue culture flask, dish or well may be used.

Generation of MCFC from EBs typically takes about 4-8 days. Generation of first myeloid cells from MCFC starts after formation of the MCFC, and continuous generation is observed thereafter. Typically, harvest is started about 3-16 days after generation of MCFC. A suitable time for production of MCFC from EB and production of myeloid cells together (i.e., step a) and step b) together) is at least about 7 days, e.g., 7-20 days. The inventors could show that, from day 7-14 onwards, at least weekly harvest of macrophages from a stirred bioreactor system is possible, showing an increasing yield over time. In a 250mL bioreactor (120mL culture volume), stable production of about 2-3x10⁷ macrophages/week is possible as early as week three, which can be maintained over time for at least 5 weeks. Of course, cultivation times and conditions may be varied depending on the phenotype of the desired cells. For example, harvesting continuously or in in small intervals may lead to production of less mature cells. The harvested cells can than, optionally, as discussed later below, be subject to further maturation.

Culture in the presence of a cytokine such as IL-3 does not necessitate continuous presence of said cytokine. It is for example possible to culture the cells in the absence of IL-3 for time periods, but differentiation and, in particular, production of myeloid cells by MCFC requires presence of IL-3. Suitable amounts or IL-3 are, e.g., 10-100 ng/mL, preferably, 20-30 ng/mL (most preferably, about 25 ng/ml) IL-3.

For production of macrophages cells having a mature phenotype, M-CSF (in a suitable concentration, e.g., 40-60 ng/mL, preferably, about 50 ng/mL M-CSF), may be added at least in step b), optionally, also in step a) of the method of the invention.

In one embodiment of the method of the invention, no cytokine additional to IL-3 is added in either step a) or step b), and the produced myeloid cells are immature myeloid cells capable of further differentiation. In said case, said method further comprises cultivating said immature cells in the presence of M-CSF (in a suitable concentration, e.g., 40-100 ng/mL, preferably, about 50 ng/mL M-CSF) until macrophages are obtained.

Good results with GMP-compliant media have been seen using ROCK-Inhibitor supplemented E8 media (Stem Cell Technologies) containing 5-200ng/mL, preferably 50ng/ml bFGF, for cultivation of single iPSC as a monolayer preceeding the suspension culture, with ROCK-Inhibitor supplemented E8⁵⁰ or E6 media (Stem Cell Technologies) for suspension culture, and with X-VIVO15 media for hematopoietic differentiation supplemented with appropriate cytokines, e.g., 50 ng/ml hVEGF, 50 ng/ml hBMP4 and 20 ng/ml hSCF which, may be followed later (e.g., at day 4 of mesoderm priming) by addition of 25 ng/ml of IL-3. Subsequent hematopoietic differentiation of primed aggregates, e.g., for macrophage production, may be pursued by changing the media to 3ml of 25 ng/ml IL-3 and 50ng/ml M-CSF supplemented X-VIVO15.

Isolating the produced myeloid cells comprises purifying the produced myeloid cells, preferably, the macrophages, to a purity of at least 50% or, preferably, at least 70%, at least 80%, at least 90% or at least 95%. If monocytes or macrophages are produced, in particular, with culture in the presence of IL-3 and M-CSF, the purity of myeloid precursor cells characterized by an expression profile of CD45⁺CD11b⁺CD34⁻TRA-1-60⁻ may be, preferably, at least 80%, at least 90% or at least 95%. At least 50%, preferably, at least 60%, or 70-90% of the produced cells are CD45⁺CD11b⁺CD34⁻TRA-1-60⁻CD14⁺ monocytes. Optionally, at least 50%, preferably, at least 60%, or 70-90% of the produced cells are CD45⁺CD11b⁺CD34⁻TRA-1-60⁻CD14⁺/CD163⁺ macrophages. In one embodiment, at least 95% of the produced cells are CD45⁺CD11b⁺CD34⁻TRA-1-60⁻ and 70-90% of the produced cells are CD45⁺CD11b⁺CD34⁻ TRA-1-60⁻CD14⁺/CD163⁺. Most (i.e., more than 50%) of the other cells are myeloid precursors of macrophages. If the isolation is continuous or the harvests are performed in shorter intervals, the percentage of precursors may be higher.

The method provides a cell population comprising CD45⁺CD11b⁺CD14⁺CD34⁻TRA1-60⁻ monocytes or CD45⁺CD11b⁺CD14⁺CD163⁺CD34⁻TRA1-60⁻ macrophages, preferably, at least 50%, at least 60%, or at least 70% of said cells, which can be used in the method of the invention.

Analysis of expression of genes associated with pluripotency and activation of innate immune response confirmed efficient differentiation of iPSC into macrophage-like cells. Importantly, genes associated with macrophage function such as the toll-like receptors (TLR) 1 and 4, CD14, or components of the NF-κB signaling pathway (gene ontology (GO) Activation of innate immunity: 0002218) were significantly upregulated in iPSC-MACs and PBMC-MACs versus iPSCs. Morphological analysis after adherence and functional analysis (e.g., phagocytic uptake of latex beads and bacteria) confirm that the generated cells are monocytes or macrophages.

The macrophages obtainable from the suspension culture method of WO 2018/202881 A1 typically have a significantly higher surface expression (as determined by FACS) of CD14 and C163 and a significantly lower surface expression of HLA than macrophages obtained from PBMC. The macrophages produced in the examples were shown to be capable of producing pro-inflammatory cytokines such as IL-6, IL-8, or TNF-alpha. Accordingly, they can be considered to be more pro-inflammatory macrophages than anti-inflammatory macrophages. By addition of suitable cytokines e.g. IL-13, IL-10, IL-4, the phenotype can be redirected to anti-inflammatory macrophages. In addition, other substances, e.g. corticosteroide, may be used to induce an anti-inflammatory phenotype. Typically, no pro-inflammatory cytokines or anti-inflammatory cytokines are added before the test of the invention is carried out.

The inventors have found that the CD45⁺CD11b⁺CD14⁺CD163⁺CD34⁻TRA1-60⁻ macrophages obtainable from the suspension culture as described above or in WO 2018/202881 A1 have a unique expression profile compared to macrophages isolated according to state of the art methods. Preferably, in these macrophages, expression of at least 10 genes selected from a group consisting of DKK1, SEPP1, PITX2, COL3A1, KRT19, A 33 P3221980, CALD1, CYR61, H19, DDIT4L, FRZB, TMEM98, NNMT, NPNT, LUM, DCN, LYVE1, MGP, IGFBP3 and NUAK1 is at least 20fold upregulated in said macrophages compared to macrophages derived from PBMC, preferably, at least 50 fold, at least 200 fold, at least 400 fold or at least 1000fold. Expression of at least 12 genes, preferably, at least 15 genes or all genes selected from a group consisting of DKK1, SEPP1, PITX2, COL3A1, KRT19, A_33_P3221980, CALD1, CYR61, H19, DDIT4L, FRZB, TMEM98, NNMT, NPNT, LUM, DCN, LYVE1, MGP, IGFBP3 and NUAK1 may be at least 100fold upregulated in said macrophages compared to macrophages derived from PBMC, preferably, at least 200 fold, at least 400 fold or at least 500 fold. Optionally, expression of CYR61, DDIT4L, KRT19, DCN, LUM, COL3A1 is at least 200 fold, at least 500 fold or at least 1000 fold upregulated in said macrophages compared to macrophages derived from PBMC.

Optionally, furthermore, in these macrophages, expression of at least 10 genes selected from a group consisting of ANPEP, CDA, CRTAM, ENST00000390237, FBP1, GBP1, GNLY, HLA_DQA1, HLA DQA2, HLA_DQB1, HLA DQB2, HLA_DRA, HLA_DRB1, HLA DRB3, HLA DRB4, HLA DRB5, IL15, LY75, S1PR4, TNFAIP6 are at least 20fold downregulated in said macrophages compared to macrophages derived from PBMC, preferably, at least 200 fold, at least 400 fold, at least 500 fold or at least 1000 fold. Expression of at least 10, at least 12 or all genes selected from said group may be at least 100fold downregulated in said macrophages compared to macrophages derived from PBMC, preferably, at least 200 fold, at least 400 fold, at least 500 fold or at least 1000 fold.

Typical expression profiles are disclosed in WO 2018/202881 A1, e.g., in Table 1. It is noted that the comparison leads to particularly high differences for genes which are practically not expressed in one of the cell types compared. In that case, even very small differences in expression of the genes can lead to high differences in the comparative rate of expression. Without intending to be limited by the theory, it is believed that the shear stress in suspension culture leads to different expression of genes in the produced myeloid cells.

In one embodiment, the method of the invention further comprises obtaining the monozyte population. They can be obtained by a method of producing myeloid cells, said method comprising steps of
a) cultivating embryoid bodies in the presence of IL-3 and, optionally, M-CSF and/or GM-CSF, for a sufficient period of time to produce myeloid cell forming complexes;
b) cultivating the myeloid cell forming complexes in the presence of IL-3 and, preferably, M-CSF and/or GM-CSF for a sufficient period of time to produce myeloid cells preferably, monozytes; and
c) isolating said myeloid cells, preferably, monozytes,
d) and, optionally, freezing said myeloid cells, and thawing and washing said myeloid cells,
wherein, if the isolated cells were not cultivated in the presence of M-CSF and/or GM-CSF in steps a) and/or b), they are cultivated in the presence of M-CSF and/or GM-CSF after isolation to provide monozytes. Step a) is preferably carried out in in suspension culture. It may also be carried out in adherent culture. Step b) is preferably carried out in in suspension culture. Preferably, both step a) and b) are carried out in suspension culture.

Monocyte cell populations (either prepared in adherent culture or in suspension culture, or in a mixed form) may be frozen, e.g., according to methods known in the art. They are thawed before use. Populations isolated, e.g., from different batches from one or more cultures at one or more time points can be combined if higher cell numbers are needed, and/or populations derived from pluripotent stem cells from different subjects may be pooled.

If the monocyte population(s) is/are frozen, the method of the invention may further comprise thawing and washing the monozyte population before step (i), wherein the monozytes are optionally adapted to a cell number of about 1*10⁴ to about 5*10⁴ cells per vial for the incubation of step (i).

### Determining a reaction of the monocytes

The method of the invention comprises (ii) determining a reaction of the monozytes in the population to the presence of the at least one pyrogen. Determining the reaction of the monozytes to the presence of the at least one pyrogen may e.g., comprise
i) determining quantity of an inflammatory cytokine selected from the group comprising IL-1beta, IL-6, IL-8 and TNF-alpha, MCP-1, IL-10, IFN-alpha, IFN-beta, IFN-gamma, IFN-lambda (IFN-1ambda1, IFN-lambda2, IFN-lambda3) or a prostaglandin such as PGE2 or a High-Mobility-Group-Protein (HMGP) such as HMGB1 or neopterin expressed by the human monozytes, or
ii) determining expression of a surface activation marker expressed by the human monocytes.

The quantity of an inflammatory cytokine or a prostaglandin or a HMGP may be determined
a) by an antibody-based test in the culture supernatant from the incubation, preferably, with an ELISA,
b) by quantitave PCR,

The detection of the quantity of the inflammatory cytokine, prostaglandin or HMGP may comprise the use of a method selected from the group comprising immunodiffusion techniques, immunoe-lectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. It may be FACS-based, e.g., an intracellular FACS for at least one cytokine, or an ELISPOT assay. The person skilled in the art is familiar with these methods, which are also described in the state of the art, for example in Zane, H.D. (2001): Immunology
- Theoretical & Practical Concepts in Laboratory Medicine, W. B. Saunders Company, in particularin Chapter 14.

Preferably, the method of the invention comprises determining the quantity of an inflammatory cytokine expressed by the monozytes. An advantageous test format that can be easily standardized and automated, e.g., for routine purposes, is an ELISA such as a sandwich ELISA. The assay may also be a multiplex assay, e.g., a Luminex^{®} Multiplex Assay. For routine tests, e.g., for quality control, an ELISA for a single cytokine is typically sufficient, e.g., for IL-1beta, IL-6, IL-8 or TNF-alpha or MCP-1. IL-6, for example, can be easily determined, and it is a cytokine often determined in conventional macrophage activation tests.

Alternatively, ii) expression of a surface activation marker expressed by the human monocytes can be determined, e.g., by FACS. Surface activation markers that can be analysed include, e.g., CD80, CD86, CD11c, MHCII, CD38, CD282 and/or CD64.

It is one of the advantages of the present invention that the monocyte populations employed can be genetically modified, e.g., to allow for an easier and/or more differentiated analysis of the cellular response to the at least one pyrogen.

Thus, in one embodiment, the monozytes comprise a reporter gene that is operably linked to a regulatory element inducible by at least one pyrogen. The regulatory element can be a promotor or enhancer. The regulatory element may be, e.g., an IL-6 promotor, a TNF-alpha-promotor, an IL-1beta-promotor, an IL-8 promotor, an IFN-alpha promotor, an IFN-beta promotor and an IFN-gamma promotor, an IFN-lambda promotor (e.g., IFN-1ambda1, IFN-lambda2, IFN-lambda3), an MCP-1 promotor, an IL-10 promotor, a High-Mobility-Group-Protein (HMGP) promotor, e.g., a HMGB1 promotor or neopterin promotor, or another pro-inflammatory cytokine promotor, or a regulatory element inducible by NFκB,
wherein determining the reaction of the monozytes to the presence of the at least one pyrogen comprises determining activity of said reporter gene.

The reporter gene may e.g., be detectable colometrically. For example, it may beta-galactosidase, luciferase, a peroxidase such as horseradish peroxidase, or a fluorescent protein, such as GFP, EGFP, YFP, CFP, dsRed, eqFP611, Dronpa, TagRFPs, KFP, EosFP/IrisFP, Dendra, Katuschka red, RedStar, mTurquiose, or mCherry.

Alternatively, or in addition, the monozytes may be genetically engineered
a) to express at least one additional receptor for a pyrogen that is not present on a wild-type human monozyte, and/or
b) not to express at least one receptor for a pyrogen that is present on a wild-type human monozyte.

Such a genetic modification, in particular, a knockout (e.g., by means of CRISPR-/Cas technology) or downregulation (e.g., by means of siRNA) may allow for differentiation between different pyrogens in the test of the invention. For example, a knockout of TLR5 leads to cells that are not responsice to flagellins, so only pyrogens other than flagellin can be detected. A knockout of TLR4 leads to cells that are not responsive to LPS, so only pyrogens other than LPS can be detected. Alternatively, it may be possible to differentiate between different pyrogens based on inhibitors, or a different profile of cytokines or messengers induced.

### Kits of the invention

The invention also provides a kit suitable for testing a composition for the presence of at least one pyrogen using the method of the present invention. Such as kit may compris
(A) separately, a plurality of human monozyte populations derived from pluripotent stem cells, wherein each monozyte population is derived from one subject, or
(B) a pool of human monozyte populations derived from pluripotent stem cells of a plurality of subjects,
preferably, (A),
all subjects sharing at least one common characteristic selected from the group comprisig age, sex, ethnicity, and/or each havig a condition selected from the group comprising an allergy, an auto-immune disease, an infection, a hereditary disease and a cancer (wherein the shared characteristic preferably is not the MHC). As explained above, the use of monocytes derived from a plurality of subjects is advantageous.

The kit may further comprise, opionally, reagents for the detection of an inflammatory cytokine selected from the group comprising an antibody to said cytokine and PCR primers allowing for detection of expression of said cytokine. The kit may alternatively or additionally comprise a pyrogen standard.

### Detailed description of the drawings

**Fig. 1** Generation of monocyte/ macrophages from human iPSC and stimulation with LPS (A) Scheme the continous production of iMAC (monocytes or macrophages derived from induced pluripotent stemm cells) from hCD34iPSCclone16 (female) or hGMPDU8 (male) in suspension cultures and subsequent use in the MAT assay. (B) Release of IL-6 and other cytokines after stimulation of iMAC (hCD34iPSCclone16) with LPS (1µg/ml).

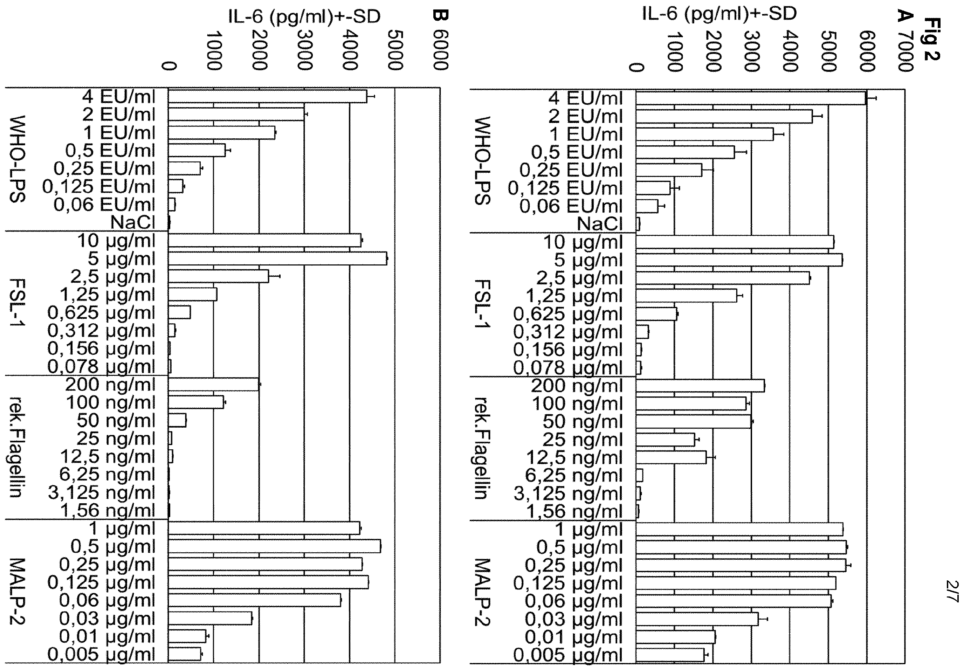

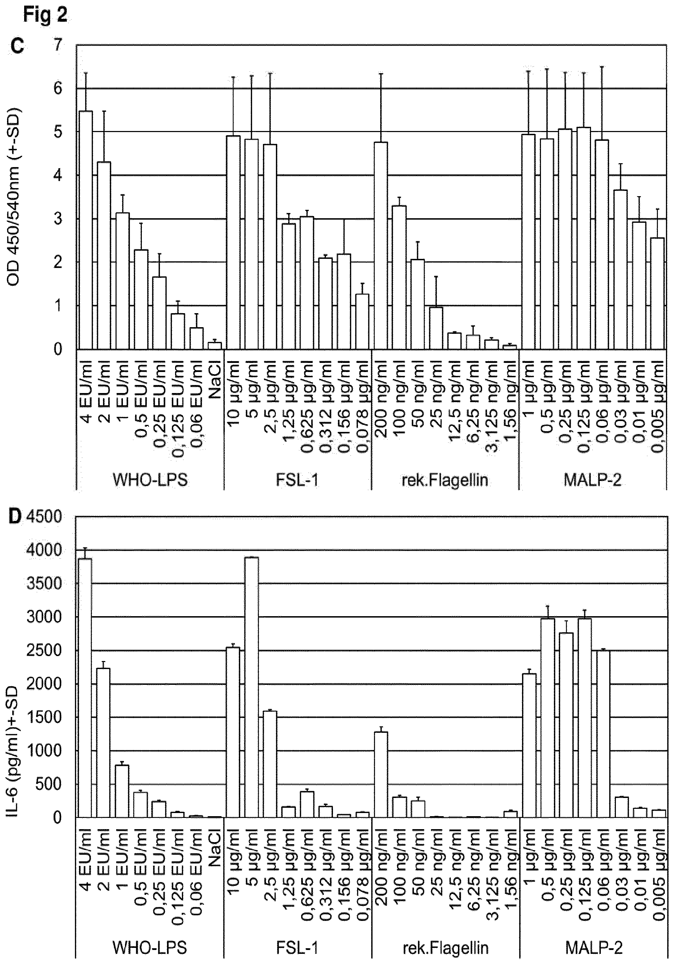

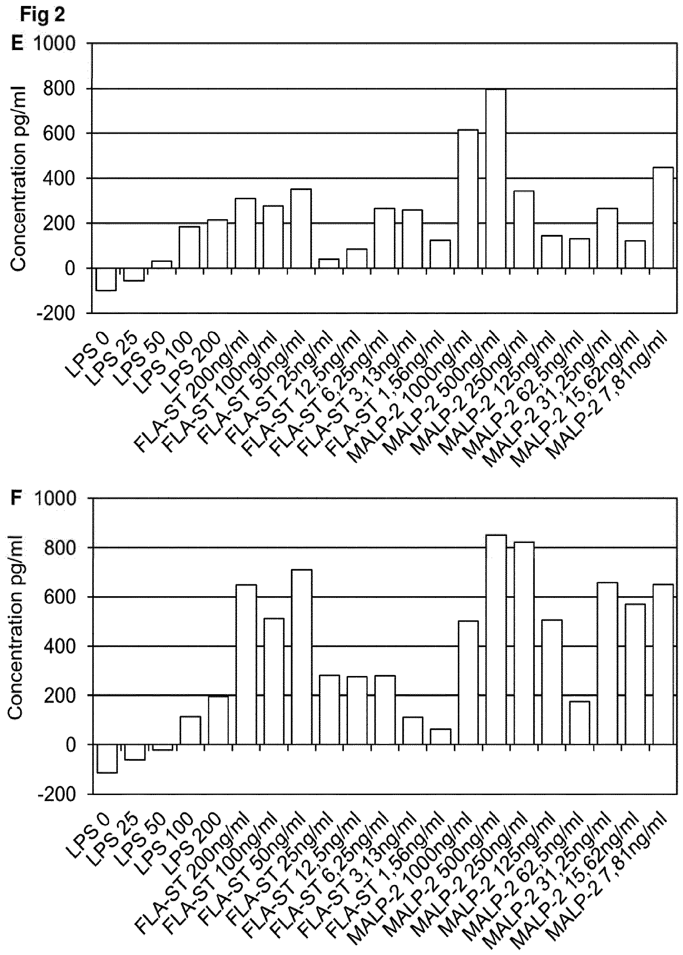

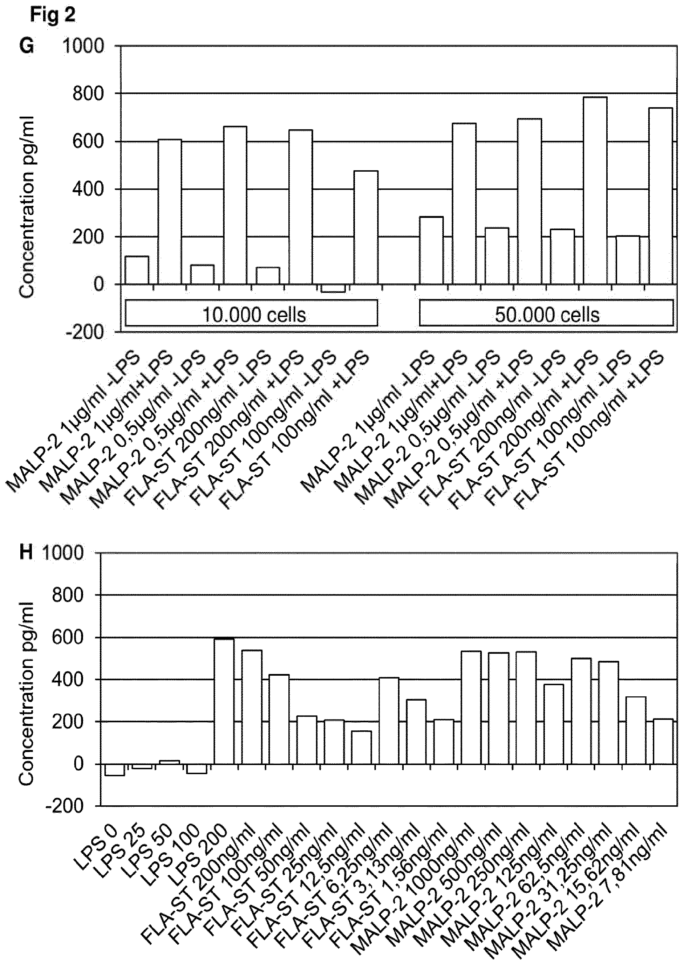

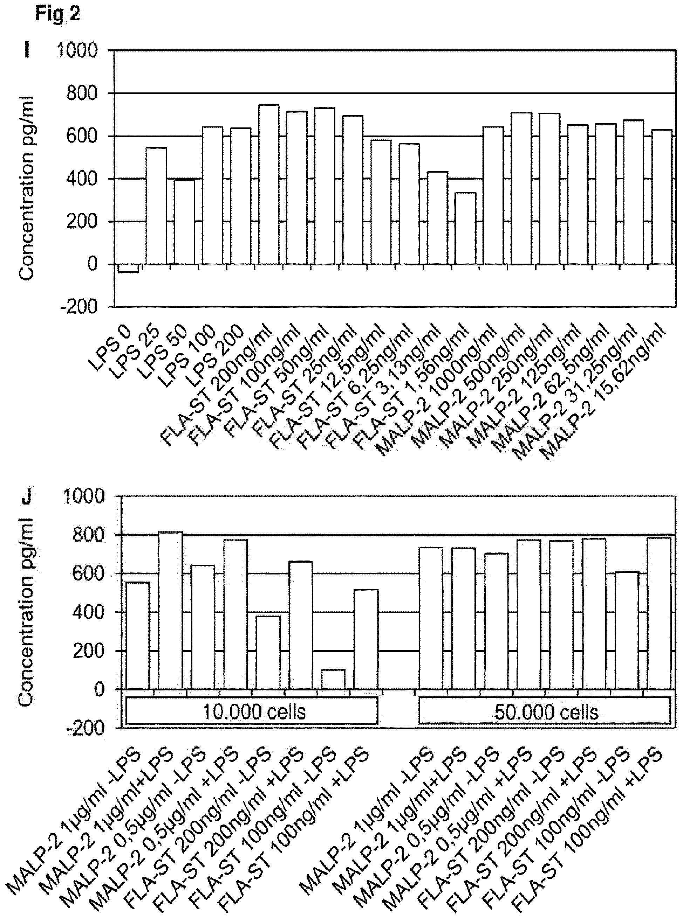

**Fig. 2** Comparison of MAT (carried out according to Example 3) with monocytes prepared from iPSC (hCD34iPSCclone16 (female) or hGMPDU8 (male)) according to the present invention (according to the method of Example 2). (A) IL-6 release after stimulation of iMAC (hCD34iPSCclone16) with LPS and NEP. 5x10^{∧}4 cells/well (differentiation into Macrophages in 96 well plate 6 days). (B) IL-6 release after stimulation of iMAC (hCD34iPSCclone16) with LPS and NEP. 1x10^{∧}4 cells/well (differentiation into Macrophages in 96 well plate 6 days). (C) IL-6 release after stimulation of iMAC (hCD34iPSCclone16) with LPS and NEP (supernatant ELISA 1:5). 5x10^{∧}4 cells/well (differentiation into Macrophages in 96 well plate 6 days). (D) IL-6 release after stimulation of iMAC (hCD34iPSCclone16) with LPS and NEP (supernatant ELISA 1:5). 1x10^{∧}4 cells/well (differentiation into Macrophages in 96 well plate 6 days). (E) TNFa release after stimulation of iMAC (hGMPDU8) with LPS and NEP. 1x10^{∧}4 cells/well (differentiation into Macrophages in 96 well plate 6 days). (F) TNFa release after stimulation of iMAC (hGMPDU8) with LPS and NEP. 5x10^{∧}4 cells/well (differentiation into Macrophages in 96 well plate 6 days). (G) TNFa release after stimulation of iMAC (hGMPDU8) +/- LPS and NEP using either 1x10^{∧}4 cells/well or 5x10^{∧}4 cells/well. (H) IL-6 release after stimulation of iMAC (hGMPDU8) with LPS and NEP. 1x10^{∧}4 cells/well (differentiation into Macrophages in 96 well plate 6 days). (I) IL-6 release after stimulation of iMAC (hGMPDU8) with LPS and NEP. 5x10^{∧}4 cells/well (differentiation into Macrophages in 96 well plate 6 days). (J) IL-6 release after stimulation of iMAC (hGMPDU8) +/- LPS and NEP using either 1x10^{∧}4 cells/well or 5x10^{∧}4 cells/well.

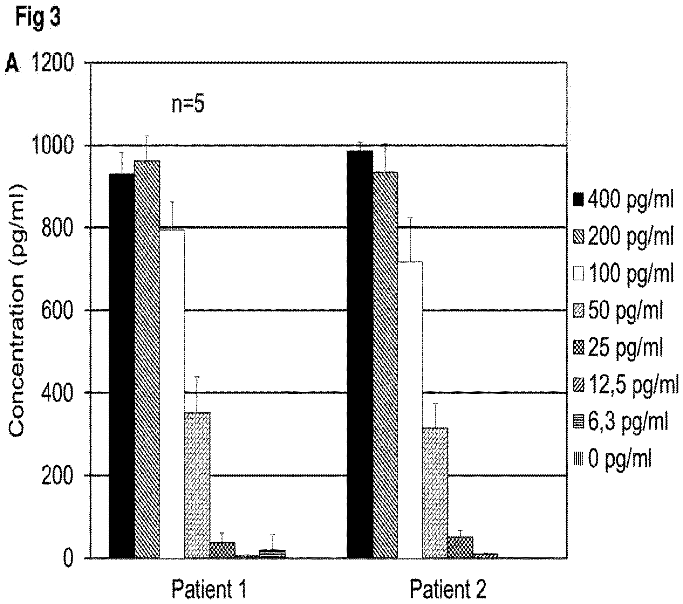

**Fig. 3** MAT (carried out similar to Example 3, but here 235µl of Medium, 10µl of Cryoblood (instead of adherent macrophages) and 15µl of sample; final volume 260 µl)) with conventional cyroblood single donors (2 donors each). Cells were incubated with LPS (from 400 pg LPS/ml down to 6.25 pg LPS/ml; 5 biological replicates of each concentration) overnight, and cytokine (IL-6) detected in the supernatant.

### Examples

### Example 1: GMP compliant suspension iPSC cultivation and hematopoietic differentiation

### Single iPSC generation:

Single iPSCs were derived directly from iPSCs cultured on murine feeder cells. iPSCs cultured on murine feeder cells were incubated with Accutase (Cell Dissociation Reagent, StemPro^{™} Thermo Scientific) for maximum 5 min in the cell culture incubator at 37°C. Accutase reaction was stopped by diluting it with either PBS or DMEM/F12 media (Thermofisher Scientific).

Pipetting up and down of resuspended cells helped to have dissociation of clumps and getting single iPSCs (not more than 3 times and very slowly). Cells were counted and subjected to monolayer cultivation on defined matrices (see next step). Alternatively, single iPSC can be derived from iPSC cultured on matrix coated dishes (e.g. GelTrex (Thermofisher Scientific), Matrigel (Corning Fisher Scientific), or Laminin (e.g. CellAdhere Laminin-521; Stem Cell Technologies) treating the cells as described before.

### Splitting cells as monolayer:

6-well tissue culture plates (e.g. NUNC plates (Thermofisher Scientific)) were coated with a matrix (e.g., GelTrex (Thermofisher Scientific), Matrigel (Corning Fisher Scientific), or Laminin (e.g. CellAdhere Laminin-521; Stem Cell Technologies) for at least one hour. Single iPSCs were seeded in the pre-coated plates in ROCK-Inhibitor (10µM) supplemented E8¹⁰⁰ or E8⁵⁰ media (Stem Cell Technologies) for further expansion. Maximum 2x10⁵ generated single iPSCs were cultured as monolayer in each well of 6-well plate. The media was changed on day 2 of and passaged at day 3 or day 4. The media was not changed the day after the seeding. These cultures were maintained for at least 10 passages.

### Aggregate formation:

5x10⁵ single iPSCs cultivated as single cells on matrix for more than two passages were inoculated in 3 ml ROCK-Inhibitor (10µM) supplemented E8⁵⁰ or E6 media (Stem Cell Technologies) in Greiner CELLSTAR multiwell culture plates (Sigma Aldrich) on an orbital shaker (70rpm) in a suspension culture for aggregate formation. Aggregate formation started within 24 hours. The media was changed at day 2, changing 2 to 2,5 ml of media. Aggregates on day 3 were transferred either for differentiation or passaged as single cell in suspension.

### Hematopoietic differentiation:

Induction of hematopoietic differentiation was started by mesoderm priming.

Mesoderm priming was started by transferring about 100 aggregates on 3 days into 3ml X-VIVO15 supplemented with 50 ng/ml hVEGF, 50 ng/ml hBMP4 and 20 ng/ml hSCF which was followed by later (at day 4 of mesoderm priming) addition of 25 ng/ml of IL3. Subsequent hematopoietic differentiation of primed aggregates was pursued by changing the media to 3ml of 25 ng/ml IL-3 and 50ng/ml M-CSF supplemented X-VIVO15. Mesoderm priming and, following that, hematopoetic differentiation were done on orbital shaker of 85 rpm.

### Example 2 Production of human pluripotent stem cell derived macrophages in adherent cultures

Alternatively, monocytes or macrophages can be differentiated in adherent culture.

IPSC colonies were disrupted to fragments using collagenase-V, and EB formation was induced by cultivation for 5 days in ESC medium supplemented with 10 ng/mL bFGF and 10 mM Rock inhibitor (Y-27632; Tocris) in six-well suspension plates on an orbital shaker (100 rpm).

After manual transfer of EBs onto tissue culture six-well plates or Bioreactor and cultivation in differentiation medium I (X-Vivo15; Lonza) supplemented with 1% penicillin-streptomycin (Life Technologies), 2 mM L-Glutamin (Life Technologies), 0,2% ß-Mercaptoethanol (Life Technologies), 25 ng/ml human IL-3 (hIL-3) and 50 ng/ml human M-CSF (hM-CSF, Peprotech) MCFCs were generated from the attached EBs within 7 days.

From d10-d15 onward, monocytes/macrophages generated by the MCFCs were harvested once a week from the supernatant and filtered through a 70µM mesh.

For further maturation, monocytes/macrophages were seeded in 96well plates (1x10^{∧}4 or 5x10⁴/well) and cultured in differentiation medium II (RPMI1640 medium supplemented with 10% fetal serum, 2 mM L-glutamine, 1% penicillin-streptomycin, and 50 ng/ml hMCSF, for 6 days.

### Example 3: Monocyte activation assay

### Use of human iPSC-macrophages for the MAT assay

▪ Use e.g. 96 well plate (flat-shape)
▪ Preparation of LPS-standard: thaw 1 aliquot at room temperature, vortex thoroughly for 5 minutes. Label 7 dilution tubes **(Borosilicate glass!)**: VV-I (= 1. pre-dilution) / VV-II (2. Pre-dilution) / 200 / 100 / 50 / 25 / 0 (all pg/ml) (LPS stock conc. 2000IU/ml).
▪ Apply dilution series:

| Tubes | LPS stock solution | carry | + NaCl-solution | |
|---|---|---|---|---|
| LPS stock solution | | | | 5 min. Vortex |
| VV-I | 10 µl | - | + 90 µl | 10 sec. Vortex |
| VV-II | - | 20 µl from VV-I | + 180 µl | 10 sec. Vortex |
| 200 | - | 90 µl from VV-II | + 810 µl | 10 sec. Vortex |
| 100 | - | 650 µl from 200 | + 650 µl | 10 sec. Vortex |
| 50 | - | 200 µl from 100 | + 200 µl | 10 sec. Vortex |
| 25 | - | 130 µl from 50 | + 130 µl | 10 sec. Vortex |
| 0 | - | - | 200µl | - |

▪Take off and discard supernatant from macrophage cultures that were seeded one week before.
▪ For the blank, apply 260µl RPMI-media + 1% PS + 10% FCS per well;
▪ For the negative control, apply 240 µl media and add 20 µl NaCl solution
▪ For the LPS-standard, apply 240 µl media and add 20 µl of each LPS soluti on/concentrati on
▪ As a sample without LPS, apply 240 µl media and add 20 µl of a positive pyrogen (e.g. Flagellin from S. typhimurium [FLA-ST](stock conc. 500µg/ml, working conc. 200ng/ml) or Macrophage-activating lipopeptide-2 [MALP-2] (stock conc. 100µg/ml, working conc. 1µg/ml))
▪ As a sample with LPS, apply 220 µl media and add 20 µl of a positive pyrogen and 20 µl of LPS (50 pg/ml dilution)
Note: now there are ∑ = **260µl** per well, 4-fold the value per concentration level.
▪ Tap plate covered with appropriate lid, so that fluids are mixed (be careful that nothing spills out!) and incubate at 37°C, ca. 5% CO2 and about 95% humidity overnight.
▪ After 22 hours (from now on it is not necessary to work pyrogen-free) take the plate out of the incubator.
▪ Take off supernatant and freeze at -20°C until usage for ELISA

### ELISA for hu IL6 / hu TNFa (R+D Duo Set)

### Plate Preparation

1. Dilute the Capture Antibody to the working concentration in PBS without carrier protein. Immediately coat a 96-well microplate with 100 µL per well of the diluted Capture Antibody. Seal the plate and incubate overnight at room temperature.
2. Aspirate each well and wash with Wash Buffer, repeating the process two times for a total of three washes. Wash by filling each well with Wash Buffer (400 µL) using a squirt bottle, manifold dispenser, or autowasher. Complete removal of liquid at each step is essential for good performance. After the last wash, remove any remaining Wash Buffer by aspirating or by inverting the plate and blotting it against clean paper towels.
3. Block plates by adding 300 µL Reagent Diluent to each well. Incubate at room temperature for a minimum of 1 hour.
4. Repeat the aspiration/wash as in step 2. The plates are now ready for sample addition.

### Assay Procedure

1. Add 100 µL of sample (pure or 1:5) or standards in Reagent Diluent, or an appropriate diluent, per well. Cover with an adhesive strip and incubate 2 hours at room temperature *(for IL6)* or overnight at 4°C *(for TNFα)*
2. Repeat the aspiration/wash as in step 2 of Plate Preparation.
3. Add 100 µL of the Detection Antibody, diluted in Reagent Diluent, to each well. Cover with a new adhesive strip and incubate 2 hours at room temperature.
4. Repeat the aspiration/wash as in step 2 of Plate Preparation.
5. Add 100 µL of the working dilution of Streptavidin-HRP to each well. Cover the plate and incubate for 20 minutes at room temperature. Avoid placing the plate in direct light.
6. Repeat the aspiration/wash as in step 2.
7. Add 100 µL of Substrate Solution to each well. Incubate for 20 minutes at room temperature. Avoid placing the plate in direct light.
8. Add 50 µL of Stop Solution to each well. Gently tap the plate to ensure thorough mixing.
9. Determine the optical density of each well immediately, using a microplate reader set to 450 nm.

Monocytes or macrophages were prepared from pluripotent stem cells, e.g., from hCD34iPSCclone16 (female) or hGMPDU8 (male), e.g., in adherent culture as described in Example 2, and subsequently used in the MAT assay as described in Example 3. The macrophages derived from induced pluripotent stem cells (iPSC) are designated iMAC herein. Release of inflammatory cytokines was determined with an ELISA. Release of IL-6 after stimulation with different cytokines is shown in Fig. 2. Fig. 2-3 shows the low variability between assays and the excellent dynamic range.

Monocytes prepared from iPSC were shown to have a higher sensitivity both with low LPS concentrations (25, 12,5, 6 pg) and in the range of < 100 pg LPS/ml than conventional monocytes prepared according to standard methods from cryoblood in the Paul-Ehrlich Institute. The improvement of the dynamic range and sensitivity leads to more options for use. Further, as the characteristics of the cells are stable, it is not required to test with every charge e.g., if is there is any sensitivity in the range > 100pg/ml).

## Claims

1. A method of testing a composition for the presence of at least one pyrogen, comprising
(i) incubating a human CD45⁺/CD11b⁺/CD14⁺/CD34⁻/TRA1-60⁻ monozyte population derived from pluripotent stem cells with said composition, and
(ii) determining a reaction of the monozytes in the population to the presence of the at least one pyrogen, preferably, determining the quantity of an inflammatory cytokine expressed by the monozytes,
wherein, preferably, the composition is (I) a pharmaceutical composition selected from the group comprising a plasma-derived pharmaceutical composition, a vaccine and another composition for injection or implantation, or (II) a composition obtained from rinsing a medical device, or the composition comprising the medical device, or (III) reference material for a diagnostic kit.

2. Use of a human monozyte population comprising CD45⁺/CD11b⁺/CD14⁺/CD34⁻/TRA1-60⁻ monozytes derived from pluripotent stem cells for testing a composition for the presence of at least one pyrogen, wherein the testing comprises carrying out the method of claim 1, wherein said testing optionally is testing for quality control and/or safety control of said composition,
wherein, preferably, the composition is (I) a pharmaceutical composition selected from the group comprising a plasma-derived pharmaceutical composition, a vaccine and another composition for injection or implantation, or (II) a composition obtained from washing a medical device, or the composition comprising the medical device, or (III) reference material for a diagnostic kit.

3. The method of claim 1 or the use of claim 2, wherein the monozyte population is derived from a plurality of subjects.

4. The method of claim 1 or the use of claim 2, wherein a plurality of human CD45⁺/CD11b⁺/CD14⁺/CD34⁻/TRA1-60⁻ monozyte populations derived from pluripotent stem cells are separately incubated with said composition, and the reaction is separately determined according to step (ii).

5. The method of any of the claims 1 or 3-4 or the use of any of claims 2-4, wherein the monozyte population is or the plurality of monozyte populations are derived from at least one healthy subject, preferably, from a plurality of healthy subjects.

6. The method of any of the claims 1 or 3-5 or the use of any of claims 2-5, wherein the monozyte population is or the plurality of monozyte populations are derived from a plurality of subjects all sharing at least one common characteristic selected from the group comprisig age, sex, ethnicity, and/or each havig a condition selected from the group comprising an allergy, an auto-immune disease, an infection, a hereditary disease and a cancer.

7. The method of any of the claims 1 or 3-6 or the use of any of claims 2-6, wherein the at least one pyrogen is
• LPS,
• a non-endotoxin pyrogen (NEP) selected from the group comprising flagellin, peptidoglycan, lipoprotein, lipoteichoic acid, FSL1, MALP2, a viral pyrogen, a yeast pyrogen and a fungal pyrogen,
• a product-related impurity,
• a process-related impurity or pyrogenic chemical agent,
• a danger-associated molecular pattern (DAMP) or
• a combination thereof.

8. The method of any of the claims 1 or 3-7 or the use of any of claims 2-7, wherein the pyrogen is a pharmaceutical composition that is to be tested for its inflammatory or anti-inflammatory potency, such as for subpotent or hyperpotent charges of a pharmaceutical composition.

9. The method of any of the claims 1 or 3-8 or the use of any of claims 2-8 wherein the monozyte population is obtainable from a method of producing myeloid cells, preferably, monozytes, said method comprising steps of
a) cultivating embryoid bodies in the presence of IL-3 and, optionally, M-CSF and/or GM-CSF, for a sufficient period of time to produce myeloid cell forming complexes;
b) cultivating the myeloid cell forming complexes in the presence of IL-3 and, preferably, M-CSF and/or GM-CSF for a sufficient period of time to produce myeloid cells preferably, monozytes; and
c) isolating said myeloid cells, preferably, monozytes,
d) and, optionally, freezing said myeloid cells, and thawing and washing said myeloid cells,
wherein, if the isolated cells were not cultivated in the presence of M-CSF and/or GM-CSF in steps a) and/or b), they are cultivated in the presence of M-CSF and/or GM-CSF after isolation to provide monozytes.

10. The method of any of the claims 1 or 3-9 or the use of any of claims 2-9, further comprising obtaining the monozyte population by a method of producing myeloid cells, said method comprising steps of
a) cultivating embryoid bodies in the presence of IL-3 and, optionally, M-CSF and/or GM-CSF, for a sufficient period of time to produce myeloid cell forming complexes;
b) cultivating the myeloid cell forming complexes in the presence of IL-3 and, preferably, M-CSF and/or GM-CSF for a sufficient period of time to produce myeloid cells preferably, monozytes; and
c) isolating said myeloid cells, preferably, monozytes,
d) and, optionally, freezing said myeloid cells, and thawing and washing said myeloid cells,
wherein, if the isolated cells were not cultivated in the presence of M-CSF and/or GM-CSF in steps a) and/or b), they are cultivated in the presence of M-CSF and/or GM-CSF after isolation to provide monozytes.

11. The method of any of the claims 1 or 3-10 or the use of any of claims 2-10, wherein the method further comprises thawing and washing the monozyte population before step (i), wherein the monozytes are optionally adapted to a cell number of about 1*10⁴ to about 5*10⁴ cells per vial for the incubation of step (i).

12. The method of any of the claims 1 or 3-11 or the use of any of claims 2-11, wherein the monocytes are CD163⁺ macrophages.

13. The method of any of the claims 1 or 3-12 or the use of any of claims 2-12, wherein in the human monozyte population, expression of at least 9 genes selected from a group consisting of DKK1, SEPP1, PITX2, COL3A1, KRT19, A 33 P3221980, CALD1, CYR61, H19, DDIT4L, FRZB, TMEM98, NNMT, NPNT, LUM, DCN, LYVE1, MGP, IGFBP3 and NUAK1 is at least 20fold upregulated compared to monozytes derived from PBMC.

14. The method of any of the claims 1 or 3-13 or the use of any of claims 2-13, wherein determining the reaction of the monozytes to the presence of the at least one pyrogen comprises
i) determining quantity of an inflammatory cytokine selected from the group comprising IL-1beta, IL-6, IL-8 and TNF-alpha, MCP-1, IL-10 or a prostaglandin such as PGE2 or a High-Mobility-Group-Protein such as HMGB1 expressed by the human monozytes
a) by an antibody-based test in the culture supernatant from the incubation, preferably, with an ELISA,
b) by quantitave PCR,
ii) determining expression of a surface activation marker expressed by the human monocytes, e.g., by FACS,
preferably, ia.

15. The method of any of the claims 1 or 3-14 or the use of any of claims 2-14, wherein the monozytes comprise a reporter gene that is operably linked to a regulatory element inducible by at least one pyrogen, wherein the regulatory element is selected from the group comprising an IL-6 promotor, a TNF-alpha-promotor, an IL-1beta-promotor, an IL-8 promotor and a regulatory element inducible by NFκB,
wherein determining the reaction of the monozytes to the presence of the at least one pyrogen comprises determining activity of said reporter gene.

16. The method of any of the claims 1 or 3-15 or the use of any of claims 2-15, wherein the monozytes are genetically engineered
a) to express at least one additional receptor for a pyrogen that is not present on a wild-type human monozyte, and/or
b) not to express at least one receptor for a pyrogen that is present on a wild-type human monozyte.

17. A kit suitable for testing a composition for the presence of at least one pyrogen using the method of any of the claims 1 or 3-16, comprising,
(A) separately, a plurality of human monozyte populations derived from pluripotent stem cells, wherein each monozyte population is derived from one subject, or
(B) a pool of human monozyte populations derived from pluripotent stem cells of a plurality of subjects,
preferably, (A),
all subjects sharing at least one common characteristic selected from the group comprisig age, sex, ethnicity, and/or each havig a condition selected from the group comprising an allergy, an auto-immune disease, an infection, a hereditary disease and a cancer, and
opionally, reagents for the detection of an inflammatory cytokine selected from the group comprising an antibody to said cytokine and PCR primers allowing for detection of expression of said cytokine, and
optionally, a pyrogen standard.
